Europäisches Patentamt

European Patent Office

Office européen des brevets

(11) Publication number: **0 094 777**

**B1**

(12) **EUROPEAN PATENT SPECIFICATION**

(45) Date of publication of patent specification: **18.03.87**  (51) Int. Cl.⁴: **G 01 N 33/543**

(21) Application number: **83302591.9**

(22) Date of filing: **09.05.83**

(54) Biologically active composition and immunoassay using the same.

<table>
<tr><td>(30) Priority: <b>10.05.82 JP 76678/82</b></td><td>(73) Proprietor: <b>FUJIREBIO KABUSHIKI KAISHA also trading as FUJIREBIO INC.</b><br><b>6-7, Shimoochiai 4-chome Shinjuku-ku</b><br><b>Tokyo 161 (JP)</b></td></tr>
<tr><td>(43) Date of publication of application:<br><b>23.11.83 Bulletin 83/47</b></td><td></td></tr>
<tr><td>(45) Publication of the grant of the patent:<br><b>18.03.87 Bulletin 87/12</b></td><td>(72) Inventor: <b>Kasahara, Yasushi</b><br><b>310, 4-4, Nagayama 3-chome</b><br><b>Tama-shi Tokyo (JP)</b><br>Inventor: <b>Suzuki, Hiromasa</b><br><b>36-12, Shimizucho</b><br><b>Itabashi-ku Tokyo (JP)</b><br>Inventor: <b>Ashihara, Yoshihiro</b><br><b>2-402, Fuchudanchi 28-1, Harumicho 1-chome</b><br><b>Fuchu-shi Tokyo (JP)</b></td></tr>
<tr><td>(84) Designated Contracting States:<br><b>DE FR GB IT NL</b></td><td></td></tr>
<tr><td>(56) References cited:<br><b>GB-A-2 019 562</b><br><b>US-A-4 134 792</b></td><td>(74) Representative: <b>Silverman, Warren et al</b><br><b>HASELTINE LAKE & CO. Hazlitt House 28</b><br><b>Southampton Buildings Chancery Lane</b><br><b>London WC2A 1AT (GB)</b></td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a method of measuring antigen and antibody and to a biologically active composition for use therein.

Various methods of measuring antigen and antibody have been developed. Of these methods, enzyme immunoassay has become widely employed because it has been found to be superior to other methods in its specificity, sensitivity, simplicity of operation and simplicity of apparatus requirements. Nevertheless attempts have been investigated in order to simplify further the operation of this method and raise its sensitivity.

In a competitive method described in GB—A—2019562 for determining the presence in a sample of an analyte which is a member of a specific binding pair consisting of a ligand and its homologous receptor, use is made of discrete dispersible solid particles to which are conjugated one of the members of the pair and a member of a signal producing system. When the sample and the remaining member of the pair conjugated to a signal label are added, channelling effect occurs on the surface of the particles, due to the proximity of all the components.

It is an object of the present invention to provide an enzyme immunoassay method which possesses improved simplicity and improved sensitivity and a biologically active composition for use therein.

According to one aspect of the invention, there is provided a biologically active composition which comprises discrete areas of immobilization on a single polymer at different locations, there being at least one area on which an antigen or antibody is immobilised and at least one area on which an enzyme, enzyme inhibitor or enzyme activator is immobilised.

In a second aspect, the invention provides a composition which comprises two polymer materials bonded to each other, one polymer material having an antigen or antibody immobilised thereon and the other having an enzyme, enzyme inhibitor or enzyme activator immobilised thereon.

This invention is applicable to antigens and antibodies in general, and the antigen and antibody may be selected according to the use to which the biologically active composition is to be put. Among antigens which may be used are haptens and first antibodies in the case of the double antibody method. The antibodies include the digestion products of an immunoglobulin with protease, such as F(ab')$_2$ Fab' and Fab, and second antibodies as against first antibodies. Moreover, in the case of the double antibody method, any combination of an antigen, a first antibody and a second antibody may be used as a combination of the antigen and the antibody of the present invention. Such antigens and antibodies include the antigens and the antibodies existing in organs, blood and urine, including hormones derived from various endocrine glands, proteins in blood serum such as immunoglobulins, albumins and ferritins, immune complexes, pathogens such as HB antigen, complements, drugs such as digoxin, cyclic nucleotides, α-fetoproteins, and carcinoembryonic antigens.

The enzyme and the enzyme inhibitor or activator react with each other and are not used in combination. Examples of such combinations include α-amylase and an amylase inhibitor, v-galactosidase and an isoflavonoid, and an esterase and a bestatin.

Since the antigen or the antibody on the one hand and the enzyme or the enzyme inhibitor or activator on the other hand are immobilized on one or more carrier materials, there are four kinds of combinations to be considered, namely the antigen and the enzyme, the antigen and the enzyme inhibitor or activator, the antibody and the enzyme, and the antibody and the enzyme inhibitor or activator.

Such a biologically active composition is characterized by the separate immobilization of the antigen or the antibody from the enzyme or the enzyme activator or inhibitor. The effect of this, as will be described later, is that when the antigen or antibody, or enzyme or enzyme inhibitor or activator, reacts and combines with one immobilization material, it cannot combine with another immobilization material. However, of course, these components may react with different immobilization materials at a boundary region therebetween. The same polymer can generally be used for immobilization of both partners of the combination at different locations of an area of immobilization thereof. However, it is also possible to use two polymer materials which are bonded to each other for example using adhesive or any other bonding technique which is practicable such as welding, an antigen or an antibody being immobilized on one polymer and an enzyme or an enzyme inhibitor or activator being immobilized on the other. Moreover, with relatively large polymer particles, for example, having a diameter of more than 3 mm, since the contacting area between the particles is small in comparison with the total surface area, the above biologically active composition may consist of a mixture of two particle groups with an antigen or an antibody being immobilized on the particles belonging to one group, and an enzyme or an enzyme inhibitor or activator being immobilized on the particles belonging to the other group.

Insofar as it is possible to use a polymer material on which the two kinds of biologically active substances are immobilized at different locations on the same polymer, this polymer material may be a block-copolymer or a graft-copolymer. In this case, an antigen or an antibody and an enzyme or an enzyme inhibitor or activator are separately immobilized by using the different functional groups of the above copolymer. —NH$_2$ —COOH, —CHO, —OH and —SH may be used as the functional groups.

When two polymer materials are used which are bonded together, the immobilizations may be carried out before or after the bonding.

The shape which the polymer material assumes may be a sphere, a disc, a cube or a ring.

The immobilization procedure itself may be carried out according to any convenient method. Those biologically active substances which are to be used which are proteins may be immobilized by using a convenient method for immobilizing biologically active proteins such as enzymes. For example, there may be used a covalent bonding method such as the diazotization method, the peptide synthesis method or the alkylation method, the ionic bond method or the physical adsorption method. When materials other than proteins are to be bonded, it is necessary to consider the functional groups of the biologically active substance and of the carrier material. The immobilized antigen may be constituted by the reaction product of an antigen and a first antibody, the antigen having previously been covalently bound to a carrier material and then the reaction product may be further allowed to react with a second antibody.

When an antigen is to be measured by using a biologically active composition of the invention, an antibody capable of reacting with the antigen to be measured or an antigen capable of reacting with such an antibody is immobilized on a carrier material to form one immobilization region. On the other hand, when an antibody is to be measured, an antigen capable of reacting with the antibody to be measured or an antibody capable of reacting with such an antigen is immobilized.

The conjugate used in carrying out the method of this invention may comprise (a) an antibody or an antigen capable of reacting with the immobilized antigen or antibody respectively, as the case may be, of the biologically active composition of this invention and (b)(i) an enzyme inhibitor or activator or (b)(ii) an enzyme capable of reacting with (c)(i) the immobilized enzyme or (c)(ii) enzyme inhibitor or activator of the biologically active composition of this invention, and the antigen or the antibody to be measured is contacted with the above composition in an aqueous solution. A reaction between the antigen and the antibody then competes with a reaction between the enzyme and the enzyme inhibitor or activator. The antigen or antibody can subsequently be determined by measurement of enzyme activity which is present.

The antigen or antibody to be measured thus need not be the subject antigen or antibody whose determination is required. For example, if the double antibody method is carried out, the reaction product of the subject antigen to be measured and a first antibody is the antigen to be measured in the foregoing procedure.

As will be appreciated from the foregoing, the conjugate may be made up of any one of four pairings: an antigen and an enzyme; an antigen and an enzyme inhibitor or activator; an antibody and an enzyme; and an antibody and an enzyme and an enzyme inhibitor or activator. Moreover, this conjugate should react with both immobilization regions of the biologically active composition, and accordingly, the choice of pairing is determined according to the biologically active composition.

When both materials to be combined are proteins, the conjugate may be produced by the cross-linking method or by peptide synthesis, which methods are suitable for immobilizing biologically active protein such as an enzyme. Should one or both materials not be a protein, a method of producing the conjugate may readily be selected in accordance with the functional groups of the materials to be combined.

The principle underlying the measurement of antigen or antibody according to the method of the present invention may be illustrated by reference to the case in which an antigen is measured by using a biologically active composition where an antigen and an enzyme inhibitor are immobilized and a conjugate comprising an enzyme and an antibody. If the antigen to be measured is absent, the conjugate combines only with the immobilized antigen and does not combine with the immobilized enzyme inhibitor, because the binding force between antigen and antibody is usually stronger than the binding force between enzyme and enzyme inhibitor. On the other hand, when the antigen to be measured is present, the antigen to be measured and the immobilized antigen compete for reaction with antigen and antibody of the conjugate. When the conjugate combines with the antigen to be measured, the enzyme portion of the conjugate is now able to react with the immobilized enzyme inhibitor of the biologically active composition. As a result, the enzyme activity of the conjugate is reduced and the amount of the antigen to be measured can be determined by measuring the extent to which the enzyme activity of the conjugate has been lowered and then referring to a calibration curve indicating the relationship between the amount of liberated antigen and the lowering % of enzyme activity which has previously been measured.

The measurement of antigen or antibody is carried out by contacting the biologically active composition with the conjugate and with the antigen or the antibody to be measured in an aqueous solution. The order of contacting is immaterial. The biologically active composition may be contacted with the conjugate and the antigen or the antibody simultaneously of the biologically active composition may first be contacted with either of the conjugate or the antigen or the antibody and then contacted with the remaining one. The measurement may also be carried out in such a manner that a conjugate comprising an antigen and an enzyme is allowed to react with an antibody immobilized on the wall of a tube and then the unreacted conjugate is scavenged by an enzyme inhibitor immobilized on a bead.

The aqueous solution reaction medium employed in the method of this invention is preferably kept at a pH suitable for the reaction of antigen and antibody and for the reaction of enzyme and its inhibitor or activator, and accordingly, the use of a buffer solution, for example a phosphate buffer solution, a borate solution or a tris-HCl buffer solution is preferable. The pH chosen depends on the nature of the antigen, the antibody, the enzyme and the enzyme inhibitor or activator, and is usually in the range of pH 5 to 8. The temperature of the aqueous solution will also be selected according to the nature of the antigen, the antibody, the enzyme and the enzyme inhibitor or activator, and is usually in the range of from 15 to 45°C.

After the contacting of the various materials with each other the enzyme activity or the enzyme inhibitory or activating activity of the biologically active composition or of the aqueous solution is measured. The measurement may be carried out by an convenient method, for example colourimetry, fluorometry, and emission spectrography using a photon counter.

The assay method of the present invention may be used to detect a wide variety of antigens and antibodies relatively simply. Of conventional methods, the sandwich method is one which, among enzyme immunoassay methods, has the defect of complicated operation. The homogeneous enzyme immunoassay has the defect of insufficient sensitivity. In contrast, the method of the invention is simple to operate and possesses sufficiently high sensitivity. The method of this invention has been made possible by both the antigen or the antibody and the enzyme or the enzyme inhibitor or activator being immobilized with the immobilization phases being separated from each other. As a result of this phase separation, the conjugate of antigen or antibody and enzyme or enzyme inhibitor or activator which has combined with one immobilization phase cannot combine with the other immobilization phase. Accordingly, high sensitivity can be achieved in spite of such simple operation.

The following examples illustrate this invention:

Example 1
(1) Preparation of biologically active composition
10 grams of dextran having an average molecular weight of 500,000 dalton (manufactured by Pharmacia Fine Chemicals) were dissolved in 50 ml of 0.1 N NaOH containing 80% ethanol. 0.6 g of sodium monochloroacetate was gradually added to the solution, and allowed to react at 30°C for 5 hours to introduce methylene carboxylic groups (more commonly known as carboxymethyl groups) to the dextran.

After the reaction, a part of the reaction product was dialysed against 10 mM borate buffer solution of pH 6.0, and 100 spherical nylon beads, 6 mm in diameter, were added to the dialysate, the amino groups of nylon beads having previously been liberated by treating with 3N HCl for 24 hours. Water-soluble carbodiimide (hereinafter referred to as WSC) was added to the mixture, and allowed to react at 4°C for 6 hours. In this way, the dextran became partially reacted with the amino groups of the nylon beads.

The beads were washed twice with the same buffer solution described above.

Amino-substituted phenylacetoaldoxime which is the inhibitor of β-galactosidase, was dissolved in 10 mM borate buffer solution pH 5.5, and allowed to react with succinic anhydride. The reaction product was added to the above washed beads in a concentration of 1 mg/ml, and allowed to react at 30°C for 18 hours in the presence of WSC. This inhibitor reacted with the remaining amino groups of the nylon beads.

0.02 M sodium periodate solution was added to the nylon beads, and allowed to react at 4°C for 30 minutes. Subsequently, ethylene glycol was added in a concentration of 0.04 M, and allowed to react at room temperature for 1 hour. The reaction solution was removed, and 10 g/ml of ferritin dissolved in 50 mM sodium carbonate buffer solution pH 8.5 were added to the nylon beads, and allowed to react at ambient temperature for 4 hours. 1 mg of sodium borohydride was added to the reaction mixture, and allowed to stand overnight. The nylon beads were then washed twice with the same buffer solution containing 0.5% bovine serum albumin (BSA), and preserved in 20 mM sodium phosphate buffer solution pH of 7.5.

(2) Preparation of conjugate
β-D-Galactosidase prepared from hog liver was combined with antibody by the conventional maleimide crosslinking method.

20 ng of the IgG fraction of goat, anti-human ferritin antibody were dissolved in 1 ml of 0.1 M sodium acetate of pH 4.5. 0.4 ng of pepsin was added to the solution, and the above antibody was digested at 37°C for 16 hours. The pH of the digest was adjusted to 8.0, and the digest was introduced into a column of Sephadex G-150 (Sephadex is a Registered Trade Mark). Then the adsorbates were eluted using 0.1 M sodium borate buffer solution of pH 8.0, and the F(ab')₂ fraction was separated.

This fraction was allowed to react with 20 µmol of 2-mercaptoethylamine in 0.1 M sodium acetate buffer solution of pH 5.0 at 37°C for 90 minutes, and the excess reagent was removed by passage through a column of Sephadex G-25.

Fab' thus produced was added to 0.1 M sodium acetate buffer solution of pH 5.0. 1.0 ml of a saturated aqueous solution of N,N'-(1,2-phenylene)bismaleimide was added to the Fab' solution, and the mixture was allowed to react at 30°C for 20 minutes. After the reaction, maleimide-Fab' was collected by using a Sephadex G-25 column.

20 µl of β-D-galactosidase solution containing ammonium sulphate in a suspended state was added to the maleimide-Fab', and allowed to react at 30°C for 20 minutes. The reaction mixture was neutralized and purified by passage through a Sepharose 6B column (Sepharose is a Registered Trade Mark) equilibrated with 10 mM sodium phosphate buffer solution of pH 7.0 and containing 0.1% BSA-1 mM $MgCl_2$—1 M NaCl to produce an enzyme-labelled antibody.

(3) Measurement of Ferritin Antigen

One of the nylon beads of the biologically active composition prepared in the foregoing stage (1) was placed in a test tube, and 0.4 ml of 0.1 M phosphate buffer solution pH 7.0 was added. Subsequently, 50 µl of a ferritin standard solution or a diluted blood serum and 50 µl of diluted enzyme labelled antibody prepared in the paragraph (2) were added to the contents of the test tube, and allowed to react at 37°C for 1 hour. 0.5 ml of 0.005 M p-nitrophenyl β-D-galactopyranoside was added as the substrate, and allowed to react at 37°C for 15 minutes. 2.0 ml of 0.4 M glycine-NaOH buffer solution (pH 10.5) were added to the reaction solution, and the reaction was stopped. Then, the absorbance of the reaction solution at 400 nm was measured.

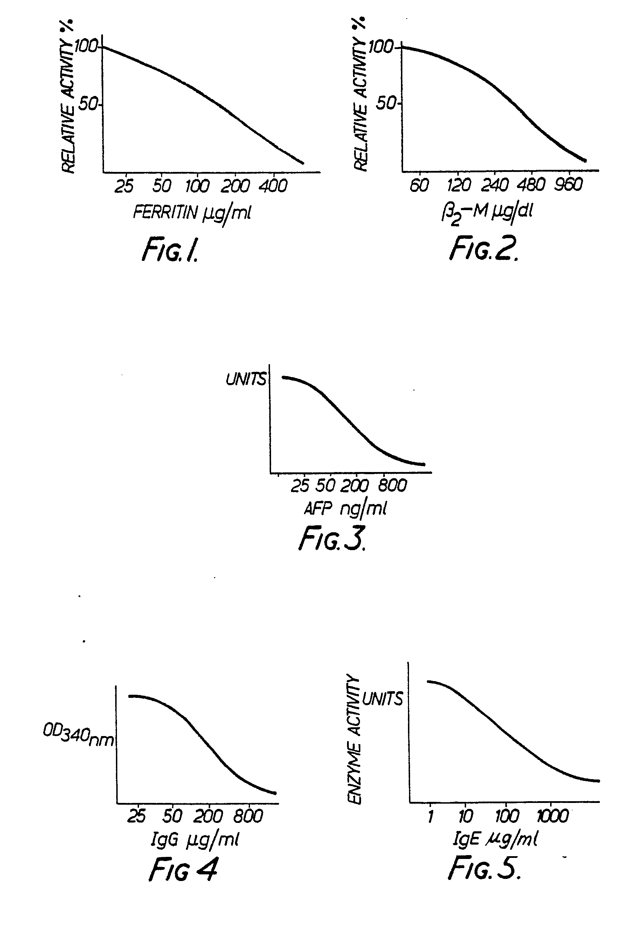

The results of the relationship between the concentration of ferritin and the relative activity of β-D-galactosidase of the reaction solution are shown in Figure 1 of the accompanying drawings. When the ferritin concentrations of various blood sera were measured by using the curve of Figure 1 as the calibration curve with the foregoing experimental procedure a good correlation between the results of this method and the results obtained by the usual method (the RIA method) was obtained.

Example 2

(1) Preparation of Biologically active composition

0.5 g of a cation-exchange resin (Amberlite IRC-50) and 0.6 g of an anion-exchange resin (Amberlite IR-45) were separately ground (Amberlite is a Registered Trade Mark). The powders were uniformly spread on separate polystyrene plates having an area of about 1 cm², and adhered thereto by pressing at 400 kg/cm².

This cation-exchange resin plate was immersed in dioxane, and 0.1 M N-hydroxysuccinimide and N,N'-dicyclohexylcarbodiimide were added to react thereon at ambient temperature for 90 minutes with stirring. The plate was washed with a small amount of methanol, and 2.5 ml of β₂-microglobulin solution diluted with 0.1 M $NaHCO_3$ solution were added. Then, the mixture was allowed to react at 4°C for 24 hours. The plate was washed with 0.05 M tris buffer solution pH 8.0, and as a result an immobilized β₂-microglobulin plate was obtained.

The anion-exchange resin plate was immersed in 50 mM sodium borate buffer solution of pH 6.0 containing 10 mg/ml of aminopeptidase B prepared from a rat liver, and WSC was added. The mixture was allowed to react at ambient temperature for 2 hours. The plate was washed with the same buffer solution, and as a result, an immobilized enzyme plate was obtained.

(2) Preparation of conjugate

The N-Hydroxysuccinimide ester of bestatin which is the inhibitor of aminopeptidase B and which has been isolated from an Actinomycetes culture was dissolved in 0.1 M $NaHCO_3$ in a concentration of 0.02 mg/ml. 10 mg of an IgG fraction of goat anti-human β₂-microglobulin were added to the solution, and allowed to react at ambient temperature for 2 hours. The reaction mixture was then separated by using Sephadex G-25 column, (Sephadex is a Registered Trade Mark), and an inhibitor labelled antibody was obtained.

(3) Measurement of β₂-Microglobulin

The immobilized β₂-microglobulin plate from stage (1) was placed in a test tube, and 0.4 ml of 50 mM sodium phosphate buffer solution of pH 7.2 containing the inhibitor labelled antibody and in different runs, 0.1 ml of β₂-microglobulin standard solution or a diluted blood serum specimen was added. The mixture was then allowed to react at 37°C for 30 minutes. 0.5 ml of L-arginine-β-naphthylamide aqueous solution was added as the substrate, and allowed to react at 37°C for 30 minutes. 1.0 ml of a garnet reagent was added, and the reaction solution was allowed to stand at ambient temperature for 15 minutes. Then, the absorbance of the reaction solution at 525 nm was measured.

A calibration curve showing the relationship between the concentration of β₂-microglobulin and the relative activity of aminopeptidase B in the reaction solution is shown in Figure 2 of the accompanying drawings.

Example 3

(1) Preparation of biologically active composition

20 mg of a proteinaceous amylase inhibitor obtained from wheat flour and having a molecular weight of about 24000 were dissolved in 0.1 M phosphate buffer solution pH 7.5 containing 5 mM EDTA. 100 µl of a solution containg 9 mg/ml of S-acetylmercaptosuccinic anhydride in dimethylsulphoxide were added to the inhibitor solution which was warmed at 37°C for 1 hour. 110 µl of 1 M hydroxylamine of pH 7.5 were then added, and reaction was effected at 37°C for 30 minutes. The reaction mixture was introduced into a column of Sephadex G-25, and gel filtration was carried out using 0.1 M phosphate buffer solution of pH 6.5 containing 1 mM EDTA to remove unreacted S-acetylmercaptosuccinic acid and accordingly a solution of an amylase inhibitor into which mercaptyl groups had been introduced was obtained.

Separately, spherical nylon beads of 4 mm in diameter, were contacted with a 3N $NH_4Cl$ solution for 24 hours to liberate their amino groups. The treated nylon beads were washed with water, and immersed in a 0.1 M phosphate buffer solution of pH 6.3. A volume of a solution of 2 mg of 4-maleimidomethyl-cyclohexane-1-carboxylic acid succinimide ester (CHMS) per ml of dioxane solution representing one tenth of the bead volume was then added and reaction at ambient temperature was allowed to take place for 3 hours. After the reaction, the beads were washed twice with a 0.1 M phosphate buffer solution of pH 6.3, and the above solution of an amylase inhibitor into which mercaptyl groups had been introduced was added to the washed beads, and allowed to react at 4°C overnight. The beads were washed with the same buffer solution as above, and placed in a 50 mM phosphate buffer solution of pH 7.0 containing 1% BSA to obtain immobilized amylase inhibitor beads.

Next, spherical polystyrene beads, 3.6 mm in diameter, were washed with water, and immersed in a solution of goat anti-human α-fetoprotein specific IgG ($OD_{280}=0.1$) dissolved in a 20 mM phosphate buffer solution of pH 7.5. The mixture obtained was allowed to stand at 4°C for 2 days, and the IgG was absorbed on the beads. The beads were sufficiently washed with a 20 mM phosphate buffer solution of pH 7.5, and preserved in a 50 mM phosphate buffer solution of pH 7.5 containing 1% BSA and 0.14 M sodium chloride to obtain immobilized antibody beads.

(2) Preparation of conjugate

5 mg of α-amylase obtained from hog pancreas were dissolved in a 0.1 M phosphate buffer solution of pH 7.5 containing 5 mM EDTA. 100 μl of a solution of 9 mg S-acetylmercaptosuccinic anhydride per ml of dimethylsulphoxide were added to the solution, and warmed at 37°C for 30 minutes. Subsequently, 110 μl of 1 M hydroxylamine of pH 7.5 were added, and reaction was allowed to take place at 37°C for 30 minutes. This reaction mixture was introduced into a column of Sephadex G-25, and gel filtration was carried out by using a 0.1 M phosphate buffer solution of pH 6.5 containing 1 mM EDTA to remove unreacted S-acetylmercaptosuccinic anhydride. A solution of α-amylase into which mercaptyl groups had been introduced was then obtained and was then concentrated to 1 ml.

Separately, 1 mg of α-fetoprotein (AFP) obtained from human ascites was dissolved in 1 ml of a 0.1 M phosphate buffer solution of pH 6.3. 100 μl of a 2 mg/ml CHMS-dioxane solution were added, and reaction was allowed to occur at ambient temperature for 1 hour. The reaction mixture was then introduced into a column of Sephadex G-25 (1 cm×40 cm), and gel filtration was carried out by using a 0.1 M phosphate buffer solution of pH 6.5 containing 1 mM EDTA to remove unreacted CHMS. The reaction product of 4-maleimidomethyl-cyclohexane-1-carboxylic acid (CHM) and human AFP solution thus produced was concentrated to 1 ml. The αm-amylase into which mercaptyl groups had been introduced was added to this concentrate, and reaction was allowed to occur at 4°C overnight. The reaction mixture was separated by gel filtration using Sephacryl® S-200 column (1 cm×120 cm), and the AFP-amylase conjugate was obtained.

(3) Measurement of AFP

One immobilized amylase inhibitor bead and one immobilized antibody bead was placed in each test tube, and 800 μl of 50 mM tris buffer solution—0.14 M NaCl—0.2 M BSA of pH 7.8 were added.

AFP solution having a concentration of 800 mg/ml was prepared, and the solution was diluted to produce 4n diluted solution series.

Each 100 μl of the diluted solution and 100 μl of the AFP-amylase combination material were added to each test tube, and allowed to stand at ambient temperature for one and one half hours.

Subsequently, 1 ml of "Autopack α-amylase" (trade name, manufactured by Boehringer Mannheim GmbH) was added as substrate, and the absorbance at 405 nm of the reaction solution after 10 minutes was measured.

The calibration curve showing the relationship between the AFP concentration and the enzyme activity is shown in Figure 3 of the accompanying drawings.

Next, various human sera were diluted 5 times, and measurements were carried out in the same manner as above using, each time 100 μl of the diluted sera. The AFP concentrations of each serum were determined by using the curve of Figure 3 as the calibration curve. For comparative purposes, the AFP concentrations of the same sera were measured by the conventional radioimmunoassay (RIA) method. The results obtained are shown in the following table.

| Serum | The method of the invention | RIA method |
|-------|------------------------------|------------|
| A | 50 ng/ml | 43.2 ng/ml |
| B | 180 | 195.9 |
| C | 430 | 404.3 |
| D | 710 | 750.0 |

Example 4
(1) Preparation of biologically active composition

A plate carrier was prepared from polystyrene by the polymer blend method.

10 mg each of poly-L-cysteine and poly-L-lysine were dissolved in a dimethylsulphoxide-ether mixture solvent, and applied to the above plate. The solvent was then evaporated off under reduced pressure. A micro phase separation between a poly-L-cysteine layer and a poly-L-lysine layer formed took place on the surface of the plate owing to the difference in their condensation energies. When the SH group content of the plate was measured by the dithiopyridone method and the amino group content was measured by the ninhydrin method, the ratio of SH groups to amino groups was about 1:1. The polystyrene plate was cut into a square of 0.8 cm, and used as a carrier plate.

70 mg of bromoacetic acid and 115 mg of N-hydroxysuccinimide were dissolved in 4 ml of dioxane. 115 mg of dicyclohexylcarbodiimide were added to the solution, and reaction was allowed to occur at ambient temperature for 90 minutes. Dicyclohexyl urea which formed was removed by filtering, and a 0.1 M sodium phosphate buffer solution of pH 7.5 was added to the filtrate until the volume was 25 ml. The aforementioned carrier plate was added to the filtrate, and allowed to react at 4°C for 60 minutes. The plate was washed with 2.5 l of 0.1 M NaCl, to yield a bromoacetamide plate. This plate was immersed in a 0.1 M potassium phosphate buffer solution of pH 6.0 containing 0.01 M pyridoxal-5'-phosphate and 0.1 M DL-valine, and allowed to stand at ambient temperature for 72 hours in a dark room. The plate was then washed with 0.1 M potassium phosphate buffer solutions of pH 9.0 and pH 5.5 in turn, and immersed in the same buffer solution, but of pH 7.0, to obtain an immobilized pyridoxal-5'-phosphate plate.

5 mg of goat anti-human IgG specific IgG (α-hIgG) were dissolved in 1 ml of a 0.1 M phosphate buffer solution, pH 6.3. 100 μl of a 2 mg/ml CHMS/dioxane solution were added to the solution, and reaction was allowed to occur at ambient temperature for 1 hour. The reaction mixture was introduced into a Sephadex G-25 column and gel filtration was carried out, using a 0.1 M phosphate buffer solution of pH 6.5 containing 1 mM EDTA to remove unreacted CHMS. The CHM reaction product with α-higG thus produced was added to the above immobilized pyridoxal-5'-phosphate plate, and allowed to react at 4°C for 3 days. After the reaction, a 0.05 M phosphoric acid—0.14 M NaCl buffer solution containing 5% BSA was added to the plate, and the plate was allowed to stand at 4°C overnight in the buffer solution to obtain the biologically active composition.

(2) Preparation of conjugate

5 mg of human IgG (hIGG) were dissolved in a 0.1 M phosphate buffer solution of pH 7.5 containing 5 mM EDTA. 100 μl of a solution of 9 mg S-acetylmercaptosuccinic anhydride in 1 ml of dimethylsulphoxide were added to the solution, and warmed at 37°C for 1 hour. 110 μl of a 1 M hydroxylamine solution of pH 7.5 were added to the mixture, and reaction was allowed to occur at 37°C for 30 minutes. The reaction mixture was introduced into a Sephadex G-25 column, and gel filtration was carried out by using a 0.1 M phosphate buffer solution of pH 6.5 containing 1 mM EDTA to remove unreacted S-acetylmercaptosuccinic acid. Then, the reaction mixture was concentrated to 1 ml, and hIgG into which mercaptyl groups had been introduced was obtained.

Separately, 4 mg of G6PDH were dissolved in 1 ml of 0.1 M phosphate buffer solution of pH 6.3. 100 μl of a solution of 2 mg CHMS in 1 ml of dioxane were added to the solution, which was allowed to stand at ambient temperature for 1 hour. The solution was then introduced into a Sephadex G-25 column, and unreacted CHMS was removed by gel filtration using 0.1 M phosphate buffer solution of pH 6.5 containing 1 mM

EDTA. The above hIgG into which mercaptyl groups had been introduced was added to the CHM which had been reacted with G6PDH, and allowed to react at 4°C overnight. The reaction mixture was subjected to gel filtration by using a Sephacryl S-300 column (sephacryl is a Registered Trade Mark), and G6PDH-hIgG conjugate was obtained.

(3) Measurement of human IgG

50 μl portions of a human IgG solution having various concentrations were placed in test tubes and 450 μl portions of the above conjugate were added. Subsequently, the biologically active composition prepared in stage 1 was added to each test tube and allowed to react at ambient temperature for 1 hour. 50 μl portions of a 0.01% aqueous sodium borohydride solution were added to each test tube, and reaction was allowed to occur at ambient temperature for 20 minutes.

The biologically active composition was taken out of each test tube and washed with a saline solution. The washed composition was placed in another test tube and 1 ml of a substrate of G6PDH containing a 0.1 M glycyliglycine buffer solution of pH 8.5—20 mM MgCl$_2$, 0.5 mM G6P and 0.5 mM NADP were added to the test tube. The absorbance at 340 nm was measured.

The calibration curve produced using the results obtained is shown in Figure 4 of the accompanying drawings.

Example 5
(1) Preparation of Biologically active composition

Samples of α-amylase derived from hog pancreas and of goat anti-human IgE specifice IgG were dissolved in a 30 mM sodium citrate—0.3 M NaCl buffer solution so that the OD$_{280}$ of the solution was 0.4, and a piece of filter paper was immersed in each of the above solutions. The wet filter papers were superposed on a nitrocellulose membrane filter of 15×15 cm (made by Toyo Filter Paper Co., Ltd.), and allowed to stand at 4°C for 2 days and in this way the α-amylase and the IgG were separately adsorbed on the same individual membrane filter. Each filter thus produced was immersed in a 5% BSA—0.1 M phosphate—0.14 M NaCl solution of pH 7.5, and allowed to stand at 4°C overnight. The filters were washed with 50 mM phosphate—0.14 M NaCl buffer of pH 7.5 and dried under a vacuum at 4°C. Each dried filter was cut down in size to 0.5×0.75 cm, and stuck on both sides of an end of triacetate film 0.5×5×0.05 cm in size using a vinyl compound adhesive so that the adsorption faces were on the outside. The structure obtained was used as the biologically active composition.

(2) Preparation of conjugate

CHM was reacted with a human IgE separated from the serum of a myeloma patient using the same method as that employed in the case of AFP in Example 3, and amylase inhibitor into which mercaptyl groups had been introduced by the same method as that employed in Example 3 was added to this CHM human IgE reaction product to produce, as conjugate, IgE-amylase inhibitor. This conjugate was purified by means of gel filtration using a Sephacryl S-300 column of 2×100 cm.

(3) Measurement of IgE

50 μl samples of IgE solutions having various IgE concentrations were placed in test tubes, and 450 μl samples of a solution of the conjugate in a 50 mM phosphate—0.14 M NaCl—2% BSA solution were added to each test tube. A specimen of the biologically active composition was placed in each test tube, and reaction at ambient temperature was allowed to take place for 1 hour. After the reaction, the biologically active composition was taken out of the test tube and placed in another test tube in which 1 ml of an enzyme substrate solution ("Autopack α-Amylase", manufactured by Boehringer Mannheim GmbH), was placed and the absorbance at 405 nm after 10 minutes was measured to enable a calibration curve showing the relationship between the amount of IgE and the amylase activity to be drawn.

This curve is shown in Figure 5 of the accompanying drawings.

## Claims

1. A biologically active composition which comprises discrete areas of immobilization on a single polymer at different locations, there being at least one area on which an antigen or antibody is immobilised and at least one area on which an enzyme, enzyme inhibitor or enzyme activator is immobilised.

2. A composition which comprises two polymer materials bonded to each other, one polymer material having an antigen or antibody immobilised thereon and the other having an enzyme, enzyme inhibitor or enzyme activator immobilised thereon.

3. A composition as claimed in Claim 2, wherein said polymer materials are in particulate form, with particles of the two polymer materials being mixed together.

4. A competitive method of determining antigen or antibody by enzyme immunoassay characterised by (A), when determining antigen; contacting a biologically active composition according to any one of the preceding claims comprising, as discrete regions, (1) at least one immobilised antibody region capable of reacting with the antigen to be determined or at least one immobilised antigen region capable of reacting with said antibody and (2) at least one immobilised enzyme, enzyme inhibitor or enzyme activator region,

with a conjugate comprising (3) an antigen capable of reacting with said immobilised antibody or an antibody capable of reacting with both said immobilised antigen and the antigen to be determined and (4) an enzyme inhibitor or enzyme activator capable of reacting with a said immobilized enzyme or an enzyme capable of reacting with a said immobilised enzyme inhibitor or enzyme activator,

and with the antigen to be measured in an aqueous solution, and

measuring the enzyme activity or the enzyme inhibiting or enzyme activating activity of said biologically active composition as a measure of the antigen-content thereof;

and by (B), when determining antibody;

contacting a biologically active composition according to any one of the preceding claims comprising, as discrete regions (1) at least one immobilised antigen region capable of reacting with the antibody to be determined or at least one immobilised antibody region capable of reacting with said antigen and (2) at least one immobilised enzyme, enzyme inhibitor or enzyme activator region,

with a conjugate comprising (3) an antibody capable of reacting with said immobilised antigen or an antigen capable of reacting with both said immobilised antibody and the antibody to be determined and (4) an enzyme inhibitor or enzyme activator capable of reacting with a said immobilised enzyme or an enzyme capable of reacting with a said immobilised enzyme inhibitor or enzyme activator,

and with the antibody to be measured in an aqueous solution, and

measuring the enzyme activity or the enzyme inhibiting or enzyme activating activity of said biologically active composition or solution as a measure of the antibody-content thereof.

## Patentansprüche

1. Biologisch aktive Zusammensetzung, welche getrennte Immobilisierungsbereiche auf einem einzigen Polymer an verschiedenen Stellen enthält, wobei wenigstens ein Bereich, auf dem ein Antigen oder ein Antikörper immobilisiert ist, und wenigstens ein Bereich, auf dem ein Enzym, ein Enzyminhibitor oder Enzymaktivator immobilisiert ist, vorhanden sind.

2. Zusammensetzung bestehend aus zwei miteinander verbunden polymeren Materialien, wobei das eine polymere Material ein immobilisiertes Antigen oder einen immobilisierten Antikörper trägt und das andere in immobilisierter Form ein Enzym, einen Enzyminhibitor oder einen Enzymaktivator aufweist.

3. Zusammensetzung nach Anspruch 2, wobei die polymeren Materialien teilchenförmig sind und die Teilchen der beiden polymeren Materialien miteinander vermischt sind.

4. Verfahren zur Antigen- oder Antikörper-Bestimmung durch einen Enzym-Immuntest, dadurch gekennzeichnet, daß

(A) im Falle der Antigen-Bestimmung

eine biologisch aktive Zusammensetzung nach einem jeden der vorangehenden Ansprüche, die in getrennten Regionen (1) wenigstens eine immobilisierte Antikörperregion aufweist, die zur Reaktion mit dem zu bestimmenden Antigen fähig ist, oder wenigstens eine immobilisierte Antigenregion hat, die zur Reaktion mit dem Antikörper fähig ist, und (2) wenigstens eine Region mit immobilisiertem Enzym, Enzyminhibitor oder Enzymaktivator hat,

in Berührung gebracht wird mit einer Zurodnung, die besteht aus (3) einem Antigen, das zur Reaktion mit dem immobilisierten Antikörper fähig ist oder einem Antikörper, der zur Reaktion mit sowohl dem immobilisierten Antigen als auch dem zu bestimmenden Antigen fähig ist, und (4) einem Enzyminhibitor oder Enzymaktivator, der zur Reaktion mit dem immobilisierten Enzym fähig ist, oder einem Enzym, das zur Reaktion mit dem immobilisierten Enzyminhibitor oder Enzymaktivator fähig ist sowie mit dem im wässriger Lösung zu bestimmenden Antigen, und

die Enzymaktivität oder Enzyminhibierungswirksamkeit oder Enzymaktivierungswirksamkeit der biologisch aktiven Zusammensetzung als Maß für den vorhandenen Antigengehalt bestimmt wird und

(B) im Falle der Antikörper-Bestimmung eine biologisch aktive Zusammensetzung nach einem jeden der vorangehenden Ansprüche, die in getrennten Regionen aufweist (1) wenigstens eine immobilisierte Antigenregion, die zur Reaktion mit dem zu bestimmenden Antikörper fähig ist, oder wenigstens eine immobilisierte Antikörperregion, die zur Reaktion mit dem Antigen fähig ist, und (2) wenigstens eine Region mit immobilisiertem Enzym, Enzyminhibitor oder Enzymaktivator besitzt,

in Berührung gebracht wird, mit einer Zurodnung, die besteht aus (3), einem Antikörper, der zur Reaktion mit dem immobilisierten Antigen fähig ist oder einem Antigen, das zur Reaktion sowohl mit dem immobilisierten Antikörper als auch dem zu bestimmenden Antikörper fähig ist, und (4) einem Enzyminhibitor oder Enzymaktivator, der zur Reaktion mit dem immobilisierten Enzym fähig ist, oder einem Enzym, das zur Reaktion mit dem immobilisierten Enzyminhibitor oder Enzymaktivator fähig ist,

sowie mit dem in wässriger Lösung zu bestimmenden Antikörper,

und die Enzymaktivität oder Enzyminhibierungswirksamkeit oder Enzymaktivierungswirksamkeit der biologisch aktiven Zusammensetzung oder Lösung als Maß fü den vorhandenen Antikörpergehalt bestimmt wird.

**Revendications**

1. Composition biologiquement active qui comprend des zones d'immobilisation séparées à différentes positions sur un même polymère, dont au moins une zone dans laquelle est immobilisé un antigène ou un anticorps, et au moins une zone dans laquelle une enzyme, un inhibiteur d'enzyme ou un activateur d'enzyme est immobilisé.

2. Composition qui comprend deux substances polymères liées l'une à l'autre, une substance polymère comportant un antigène ou un anticorps immobilisé, et l'autre comportant une enzyme immobilisée, un inhibiteur d'enzyme ou un activateur d'enzyme immobilisé.

3. Composition selon la revendication 2, dans laquelle lesdites substances polymères se trouvent sous forme particulaire, les particules des deux substances polymères étant mélangées les unes avec les autres.

4. Procédé compétitif de dosage d'antigène ou d'anticorps par dosage immuno-enzymatique, caractérisé par

(A) pour le dosage d'antigène:

la mise en contact d'une composition biologiquement active selon l'une quelconque des revendications précédentes, comprenant en tant que régions séparées (1) au moins une région comportant un anticorps immobilisé, capable de réagir avec l'antigène à doser, ou au moins une région comportant un antigène immobilisé, capable de réagir avec ledit anticorps, et (2) au moins une région comportant une enzyme immobilisée, un inhibiteur d'enzyme ou un activateur d'enzyme immobilisé,

avec un conjugué comprenant (3) un antigène capable de réagir avec un dit anticorps immobilisé ou un anticorps capable de réagir à la fois avec ledit antigène immobilisé et l'antigène à doser, et (4) un inhibiteur d'enzyme ou un activateur d'enzyme, capable de réagir avec une dite enzyme immobilisée, ou une enzyme capable de réagir avec un dit inhibiteur d'enzyme ou activateur d'enzyme immobilisé, et avec l'antigène à doser dans une solution aqueuse, et

la mesure de l'activité enzymatique ou de l'activité inhibitrice d'enzyme ou activatrice d'enzyme de ladite composition biologiquement active, en tant que mesure de la teneur en antigène de celle-ci;
et par

(B) pour le dosage d'anticorps:

la mise en contact d'une composition biologiquement active selon l'une quelconque des revendications précédentes, comprenant en tant que régions séparées, (1) au moins une région comportant un antigène immobilisée, capable de réagir avec l'anticorps à doser, ou au moins une région comportant un anticorps immobilisé, capable de réagir avec ledit antigène, et (2) au moins une région comportant une enzyme immobilisée, un inhibiteur d'enzyme ou un activateur d'enzyme immobilisé,

avec un conjugué comprenant (3) un anticorps capable de réagir avec un dit antigène immobilisé ou un antigène capable de réagir à la fois avec ledit anticorps immobilisé et l'anticorps à doser, et (4) un inhibiteur d'enzyme ou un activateur d'enzyme, capable de réagir avec une dite enzyme immobilisée, ou une enzyme capable de réagir avec un dit inhibiteur d'enzyme ou activateur d'enzyme immobilisé,

et avec l'anticorps à doser dans une solution aqueuse, et

la mesure de l'activité enzymatique ou de l'activité inhibitrice d'enzyme ou activatrice d'enzyme de ladite composition ou solution biologiquement active, en tant que mesure de la teneur en anticorps de celle-ci.

FIG.1.

FIG.2.

FIG.3.

FIG 4

FIG.5.